# EUROPEAN PATENT APPLICATION

(11) **EP 1 475 444 A1**
(43) Date of publication of application: **10.11.2004**
(21) Application number: 04103240.0
(22) Date of filing: 22.04.1998
(51) Int. Cl.: C12P 37/06

(54) **Improved process for the fermentative production of penicillin**

(30) Priority: 22.04.1997 EP 97201200
(62) Divisional of application: 98921489.5
(71) Applicant: DSM IP Assets B.V., 6411 TE Heerlen (NL)
(72) Inventor: Kerkhof, Pieter Theodorus, 3191 HS Hoogvliet (NL); Kuipers, Rienk, Hendrik, 2804 KZ Gouda (NL); Walraven, Hubertus, Gerardus, Maria, 2284 TM Rijswijk (NL)
(74) Representative: Elkenbracht, Johan Christiaan

(57) **Abstract**

The present invention discloses an improved process for the preparation of 6-amino penicillanic acid (6-APA) from a fermentatively produced N-substituted penicillin, comprising the steps of extraction of the N-substituted penicillin compound as present in a fermentation broth or fluid to an organic solvent, back extraction of the N-substituted penicillin compound to water, treatment of the aqueous phase with a penicillin acylase to yield 6-APA and a side chain. The 6-APA from the thus-obtained conversion solution may be isolated by crystallization. Further improvements comprise extraction of the side chain to an organic solvent and isolation of 6-APA from the thus-obtained aqueous phase using a specific crystallization process.

## Description

### Field and background of the invention

The invention relates to a process for the preparation of penicillins, which are deacylated at the 6-amino group, by the fermentation of a penicillin producing microorganism in the presence of a side chain precursor and by deacylation of fermentatively produced N-substituted penicillin.

6-Amino penicillanic acid (6-APA) is an important intermediate in the pharmaceutical industry which is in general obtained through chemical or enzymatic cleavage ('deacylation') of penicillin G or penicillin V.

Penicillins themselves are obtained from fermentation using strains of *Penicillium chrysogenum.* In a typical penicillin G recovery process, the broth is filtered and washed, and penicillin G is extracted to butyl acetate or methyl isobutyl ketone after acidification of the filtrate. The solvent is then decolorized with aid of active carbon and the penicillin G acid is back-extracted to water upon neutralization with an aqueous potassium salt solution such as potassium acetate. This solution is admixed with sufficient butanol, the water content is reduced using evaporation of the butanol-water azeotrope, whereupon the penicillin G crystals formed are recovered by filtration and subsequent washing and drying.

In another process the crystalline material is directly obtained from the organic solution by addition of potassium acetate or other potassium salts and evaporation of the azeotrope, whereupon the crystals are filtered, washed with butanol, filtered and dried. For penicillin V the same process can be applied. However, due to better stability of penicillin V under acidic conditions, penicillin V can also be crystallized in the acid form instead of the potassium salt form, without economical unacceptable losses.

6-Amino penicillanic acid is in general obtained from a solution of dissolved solid penicillin G or V, which is then contacted with immobilized penicillin G acylase or penicillin V acylase, respectively. The conversion solution is subsequently fed to a crystallizer where the solid product is formed by precipitation at a pH of about 3.5 to 4.5. Generally, in this crystallization process the pH is lowered stepwise to avoid contamination of the final product 6-APA with high side chain levels and coloured compounds. In addition, solvents like methanol, ethanol, iso propyl alcohol, are added to avoid this unacceptable contamination. It will be clear that these types of production routes to 6-APA are lengthy and costly.

The present invention discloses an improved method for the production of 6-APA, wherein the isolation of penicillin G or V as a solid intermediate is not required and wherein the colour correction measures are made redundant.

### Description of the invention

The present invention discloses an improved process for the production of 6-amino penicillanic acid (6-APA) from a fermentation broth of a penicillin producing microorganism.

In general the novel production and purification process comprises the following steps:

A penicillin-producing microorganism is fermented in the presence of a suitable side chain precursor. The fermentation broth is filtered, whereupon the filtrate is extracted with an organic solvent to obtain an organic phase containing the major part of N-substituted penicillanic acid. Subsequently, the N-substituted penicillanic acid is back extracted to water. The aqueous phase optionally is stripped to remove traces of solvent. The remaining aqueous solution phase is enzymatically treated to yield 6-APA and a side chain, as for instance phenoxy or phenyl acetic acid.

Subsequently, 6-APA is precipitated from the aqueous conversion solution after acidification of said solution and isolated as a crystalline product. However, a more preferred embodiment of the invention comprises acidification of the conversion solution and subsequent extraction of the side chain to an organic solvent prior to precipitation of 6-APA from the aqueous phase using a pH-shift.

In the process according to the invention, the following steps are described in more detail.

A fermentation broth containing the N-substituted penicillin is obtained from any suitable fermentation process, e.g from a fermentation of a strain of *Penicillium chrysogenum* in the presence of a suitable side chain precursor, such as phenyl acetic acid or phenoxy acetic acid.

The biomass is separated from the fermentation broth using any suitable technology, such as centrifugation or filtration, yielding a penicillin-containing fermentation fluid. Preferably, a filtration step is applied to obtain said separation. The residual solids optionally are washed.

The fermentation broth or fluid is acidified to a pH within a range of 2-4, preferably to a pH within a range of 2.5-3, by addition of an acid, such as sulfuric acid, hydrochloric acid or nitric acid or a combination thereof. The N-substituted penicillin is then separated from the acidified aqueous phase by extraction to an organic solvent. The organic solvent which is used preferably is amyl acetate, butyl acetate, ethyl acetate, methyl isobutyl ketone, cyclohexanone, iso-butanol or n-butanol. The addition of a suitable de-emulsifier may improve the extraction significantly.

In one embodiment of the invention, part of the organic solvent may be added to the fermentation broth or fluid prior to acidification. This was found to be satisfactory in avoiding precipitation, said precipitation sometimes occurring as an undesired side effect of acidification of the fermentation broth or fluid. Precipitation is undesired since it will lead to loss of product and may cause subsequent operational problems.

In a next step, the N-substituted penicillin as present in the organic solvent is back extracted to water at a pH of 6-10, preferably at a pH of 7-9. The base used for pH adjustment for instance can be ammonia or an alkaline or earth alkaline hydroxide solution.

After phase separation, the aqueous phase optionally is stripped to remove the organic solvent.

Subsequently, the aqueous phase or solution is treated with a suitable penicillin acylase.

Organisms that have been found to produce penicillin acylase are, for example, *Acetobacter, Aeromonas, Alcaligenes, Aphanocladium, Bacillus* sp., *Cephalosporium, Escherichia, Flavobacterium, Kluyvera, Mycoplana, Protaminobacter, Providentia, Pseudomonas* or *Xanthomonas* species. Enzymes derived from *Acetobacter pasteurioanum, Alcaligenes faecalis, Bacillus megaterium, Escherichia coli, Providentia rettgeri* and *Xanthomonas citrii* have particularly proven to be succesful in a method according to the invention. In the literature, penicillin acylases have also been referred to as penicillin amidases.

The penicillin acylase may be used as a free enzyme, but also in any suitable immobilized form, for instance as has been described in EP 0 222 462 and WO 97/04086.

It is advantageously shown by the present invention that is is feasible to subject a fermentatively derived N-substituted penicillin compound, such as penicillin G, to an enzymatic conversion to 6-APA without the necessity for prior isolation of the N-substituted penicillin compound in a crystalline form.

The present invention also envisages the option to directly treat the fermentation fluid with penicillin acylase, i.e. without applying a prior extraction and back extraction step.

The conversion solution, resulting from the enzymatic reaction indicated above, contains 6-APA, a side chain such as phenyl or phenoxy acetic acid, coloured products and traces of unconverted product.

In one embodiment of the invention, the product 6-APA is isolated from the conversion solution by crystallization at acidic conditions. Typically, crystallization of 6-APA from an aqueous 6-APA solution is performed by adjusting the pH of the 6-APA solution to an acidic value by adding a titrant to the 6-APA solution until the pH has reached a value within a range of 2.5-4.5, preferably a value of 3-4.

In a preferred embodiment of the invention, crystallization of 6-APA from an aqueous solution is carried out by adding the 6-APA solution to a crystallization vessel which is kept at a fixed pH having a value within a range of 2.5-4.5, using a suitable titrant.

In an even more preferred embodiment of the invention, said crystallization is carried out by a stepwise adjustment of the pH of the 6-APA-solution to a final value within a range of 2.5-4.5 by adding the 6-APA solution to a series of interconnected crystallization vessels, i.e. adding the 6-APA solution to a first vessel, simultaneously adding the content of the first vessel to a second vessel, simultaneously adding the content of the second vessel to a third vessel, etc., wherein a pH range is applied in the interconnected vessels using a suitable titrant, starting at a pH in the first vessel which deviates about 0.5-2 pH units from the pH of the 6-APA solution and ending at a pH in the final vessel which has a value within a range of 2.5-4.5. Conveniently, the pH of the aqueous 6-APA solution is adjusted to the desired final value using a series of 2-6 interconnected vessels.

For instance, to obtain crystallization of 6-APA from the conversion solution, a decreasing pH range from 8 to 3 can be applied using a titrant which is an acid, such as sulfuric acid, hydrochloric acid and/or nitric acid, applying a series of 3-4 interconnected crystallization vessels.

The two preferred embodiments described above are preferably performed in a continuous mode.

In a further preferred embodiment of the invention, the phenyl or phenoxy acetic acid side chain is removed prior to crystallization of 6-APA by extraction to an organic solvent. The conversion solution is acidified and then extracted with an organic solvent, for instance amyl acetate, butyl acetate, ethyl acetate, methyl isobutyl ketone, cyclohexanone, iso-butanol or n-butanol. The acidification of the conversion solution is performed with an acid, such as sulfuric acid, hydrochloric acid or nitric acid or a combination thereof, preferably sulfuric acid, to a pH lower than 3, preferably lower than 2. It was unexpectedly found that also a high removal efficiency of the coloured impurities was obtained upon applying this extraction step.

In one embodiment of the invention, the organic solvent extraction is performed in two to four stages of intensive contact extraction, for example a combination of an intense mixer, for instance a high shear mixer with a centrifugal separation, said stages preferably operated in counter current mode.

The organic solvent, containing phenyl or phenoxy acetic acid and coloured compounds, may contain a small quantity of 6-APA. Said 6-APA may optionally be back extracted to water, preferably in two to four extraction stages, in counter current mode. The water used for this back extraction is acidified to a pH lower than 6, preferably lower than 3. The preferred water:solvent ratio is 1:5, more preferably 1:20, most preferably 1:10. The thus-obtained aqueous phase can be fed to the 6-APA crystallization or recycled into the side chain extraction.

After organic solvent extraction of the side chain, the 6-APA product can be crystallized from the aqueous phase in several ways, such as the ways which are indicated hereinabove for crystallization of 6-APA from an aqueous solution.

In a preferred mode of operation, the pH of the aqueous phase is increased to a pH having a value within a range of 2.5-5, preferably within a range of 3.5-4.5, by adding the 6-APA solution in one step to a crystallization vessel kept at the desired pH value or to a series of 2-6 interconnected crystallization vessels applying an increasing pH range. These processes can conveniently be carried out in continuous mode.

When applying this aspect of the process of the invention, the amount of side chain, e.g. phenyl or phenoxy acetic acid, and coloured compounds present in the 6-APA crystals is low as compared to conventional processes wherein the side chain is not extracted and batch mode crystallization from a neutral solution is applied.

All of the above mentioned steps, i.e. extraction, back extraction and crystallization, can be carried out in batch or fed batch mode, but because of stability reasons the preferred method is a continuous mode.

The obtained 6-APA crystals are isolated by filtration or centrifugation and dried in a conventional continuous or batch dryer.

## Claims

1. Process for the preparation of 6-amino penicillanic acid (6-APA) from a fermentatively produced N-substituted penicillin, comprising the steps of:
extraction of the N-substituted penicillin compound as present in a fermentation broth or fluid to an organic solvent, back extraction of the N-substituted penicillin compound to water, treatment of the aqueous phase with a penicillin acylase to yield 6-APA and a side chain.

2. Process according to claim 1, further **characterized in that** 6-APA is isolated from a conversion solution by crystallization.

3. Process according to claim 1 or 2, further **characterized in that** the side chain as present in the conversion solution obtained after acylase treatment is extracted to an organic solvent prior to isolation of 6-APA from the aqueous phase by crystallization.

4. Process according to claim 3, wherein the conversion solution is acidified to a pH lower than 3 prior to extraction.

5. Process according to any one of the claims 2 to 4, wherein crystallization of 6-APA from the conversion solution or aqueous phase is performed by adding the 6-APA solution to a crystallization vessel which is kept at a fixed pH having a value within a range of 2.5-4.5, using a suitable titrant.

6. Process according to any one of the claims 2 to 4, wherein crystallization of 6-APA from the conversion solution or aqueous phase is performed by adding the 6-APA solution to a series of interconnected crystallization vessels while applying a pH range using a suitable titrant, starting at a pH in the first vessel which deviates about 0.5-2 pH units from the pH of the 6-APA solution and ending at a pH in the final vessel which has a value within a range of 2.5-4.5.

7. Process according to any one of the claims 1 to 6, wherein the fermentation broth or fluid is acidified to a pH within a range of 2-4, preferably within a range of 2.5-3, prior to extraction.

8. Process according to claim 7, wherein part of the organic solvent is added to the fermentation broth or fluid prior to acidifying said broth or fluid.

9. Process according to any one of the claims 1 to 8, wherein the aqueous phase is stripped prior to treatment with a penicillin acylase.

10. Process according to any one of the claims 1 to 9, wherein the organic solvent is amyl acetate, butyl acetate, ethyl acetate, methyl isobutyl ketone, cyclohexanone, iso-butanol or n-butanol.
